# EUROPEAN PATENT SPECIFICATION

(11) **EP 3 705 895 B1**
(45) Date of publication and mention of the grant of the patent: **03.05.2023**
(21) Application number: 20161429.4
(22) Date of filing: 06.03.2020
(51) Int. Cl.: E03D 13/00, E03D 5/10

(54) **URINAL SENSOR SYSTEM**
URINALSENSORSYSTEM
SYSTÈME DE CAPTEUR D'URINOIR

(30) Priority: 08.03.2019 GB 201903162
(43) Date of publication of application: 09.09.2020
(73) Proprietor: Ideal Standard International NV, 1935 Zaventem (BE)
(72) Inventor: LAYEGHI, Kombiz, Hull, Humberside HU5 4HS (GB); DELLA-PORTA, Louis, Hull, Humberside HU5 4HS (GB); PEARSON, John, Hull, Humberside HU5 4HS (GB)
(74) Representative: D Young & Co LLP

(56) References cited:
- CN-U- 207 988 068
- GB-A- 2 201 699
- GB-A- 2 263 711
- GB-A- 2 279 086
- GB-A- 189 603 014
- US-A1- 2010 146 691

## Description

### BACKGROUND

The present invention relates particularly, but not exclusively, to a sensor system for a urinal. The invention may be used to monitor blockages in the urinal and provide an indication of these blockages or reduced flow to a user or a party in charge of maintenance. The invention may also be used in situations where it is desirable to monitor usage of the urinal or where the urine itself is desired to be monitored. Such monitoring allows optimisation of the flushing frequency and the flush timings of the urinal.

The invention further relates to a method of use of the information taken from the sensor system.

The urinal sensor system is able to accurately determine when the urinal is used and when there is a blockage, or leakage, in the urinal.

Urinal sensor systems exist in the art. Current urinal sensor systems involve locating a sensor on the back of the urinal bowl itself. Thus, in order for the sensor to detect that the urinal is in use, it is necessary that the urine contacts with the urinal bowl at the exact position in which the sensor is located. If the urine does contact the urinal at this position, the sensor system detects that the urinal is in use. If however the urine does not contact the urinal at this position, the sensor is not able to detect that the urinal is in use.

Furthermore, the urinal sensor systems designed in this way are not able to detect blockages in the outlet pipe of the urinal and are only able to detect blockages in the urinal bowl itself when the urine reaches the level of the sensor on the back of the bowl. Only at this point is the sensor able to determine that the urinal is blocked. Once this point has been reached, it is likely that the level is too high and leakage of the urine is occurred. This is unsanitary.

Therefore, there is a need to provide a urinal sensor system which is able to more accurately determine when the urinal is in use.

The inventors have devised an alternative positioning for a sensor system for the urinal. The positioning of the sensor in the present invention enables accurate monitoring of urinal usage and enables a user to be notified when the urinal is blocked. If the urinal is detected as being blocked, further flushing of the urinal is prevented in order to prevent further problems.

Furthermore, the inventors have developed a novel method of using the information from the sensor system.

Existing prior art includes GB 2,263,711, which relates to automatically flushed sanitary appliances; GB 2,279,086, which relates to apparatus for controlling the amount of water used for flushing; and GB 2,201,699, which relates to an automatically flushing urinal.

### SUMMARY OF THE INVENTION

Aspects of the invention are set out in the accompanying claims.

Viewed from a first aspect there is provided a system according to claim 1.

In some embodiments, the sensor is operable to detect the presence of fluid in the outlet pipe based on fluid contacting a portion of the outlet pipe which the sensor is connected to. In some embodiments, the sensor may be a non-contact sensor, such as an inductive sensor, or a capacitive sensor, for detecting fluid through the outlet pipe. In other embodiments, the sensor may be a pressure sensor.

In some embodiments, the at least one first signal may comprise a plurality of first signals, each first signal containing first information relating to the presence of fluid in the outlet pipe at a different point in time, wherein the processor is configured to process the first information from the plurality of first signals to generate the second signal.

In such cases, the processor may be configured to process the first information from the plurality of first signals to generate second information relating to the frequency in which the sanitary ware product is used in a period of time, wherein the second signal is generated based on the second information. Such second information can then be used to prepare usage statistics relating to the sanitary ware product.

In some embodiments, the processor may be configured to process the first information from the plurality of first signals to derive second information relating to a period of time in which fluid is present in the outlet pipe, wherein the second signal is generated based on the second information. There, the processor may be further configured to determine a blockage in the event the period of time exceeds a threshold amount of time.

In some embodiments, the second signal may comprise an instruction for controlling the operation of a light source from a sanitary ware product.

The second signal may in some embodiments comprise an instruction for controlling the operation of a flush valve from a sanitary ware product.

In some embodiments, the system may further comprise a casing which is attachable to an outlet pipe of a sanitary ware product, wherein the sensor is located inside the casing.

The system may further comprise a shroud which is configured to extend around a portion of an outlet pipe of a sanitary ware product, wherein the casing is configured to rest on a surface of the shroud for supporting the casing on the shroud.

In some embodiments, the device may be a remote location from the sensor for sending and receiving information from the sensor. The device may be operable to send information to the sensor for controlling the operation of the sensor.

In some embodiments, the device may be at least one of a computer or a handheld device.

In other embodiments, the device may be a flush valve, or a light source from a sanitary ware product.

The outlet pipe comprises an end located upstream of the sensor, wherein the end of the outlet pipe is connectable to an outlet of the sanitary ware product, and wherein the sensor is positioned directly underneath the end of the outlet pipe. By positioning the sensor directly underneath the end of the outlet pipe, and thus the outlet to which it is connected to, this increases the effectiveness of the sensor at detecting small amounts of fluid passing out from the outlet through the outlet pipe.

The flat surface may be orientated at between twenty degrees and thirty degrees with respect to the horizontal.

To allow the system to be more easily retrofitted on an existing sanitary ware product, a length of a portion of the outlet pipe from the system may be adjustable. In some embodiments, the portion of the outlet pipe may be a portion of the upstream pipe section. In other embodiments, the length may be a vertical length.

In a particular embodiment, the system may be used with a sanitary ware product in the form of a urinal.

By positioning the sensor directly underneath the outlet of the sanitary ware product, this increases the effectiveness of the sensor at detecting small amounts of fluid passing out from the outlet through the outlet pipe.

According to a second aspect, there is provided a method according to claim 16.

In some embodiments, the method may further comprise processing the second signal to indicate a status or condition of the user. The status or condition may comprise at least one of the level of hydration of the user; diabetes; and/or or a urinary tract infection.

### DRAWINGS

Aspects of the invention will now be described, by way of example only, with reference to the accompanying figures in which:
Figure 1 shows a cross section view of a sanitary ware product comprising an outlet pipe using a sensor operable to detect the presence of fluid in the outlet pipe;
Figure 2 shows a perspective view of the outlet pipe and the sensor from Figure 1;
Figure 3 shows a photograph of the sanitary ware product from Figure 1, and also shows a spreader for cleaning fluid and light source for use with the sanitary ware product.
Figure 4 shows a schematic of system showing the interaction of the sensor from Figure 1 with other components from the system; and
Figure 5 shows exemplary screenshots of a graphical user interface for use with the sensor shown in Figure 1.

While the present teachings are susceptible to various modifications and alternative forms, specific embodiments are shown by way of example in the drawings and are herein described in detail. It should be understood, however, that drawings and detailed description thereto are not intended to limit the scope to the particular form disclosed, but on the contrary, the scope is to cover all embodiments falling within the scope defined by the appended claims.

As used in this specification, the words "comprises", "comprising", and similar words, are not to be interpreted in an exclusive or exhaustive sense. In other words, they are intended to mean "including, but not limited to".

It will be recognised that the invention covers not only individual embodiments but also combinations of the embodiments that have been discussed herein, as long as such combinations fall within the scope defined by the appended claims.

### DESCRIPTION

The present teaching relates generally to a sensor system for a sanitary ware product, such as a urinal.

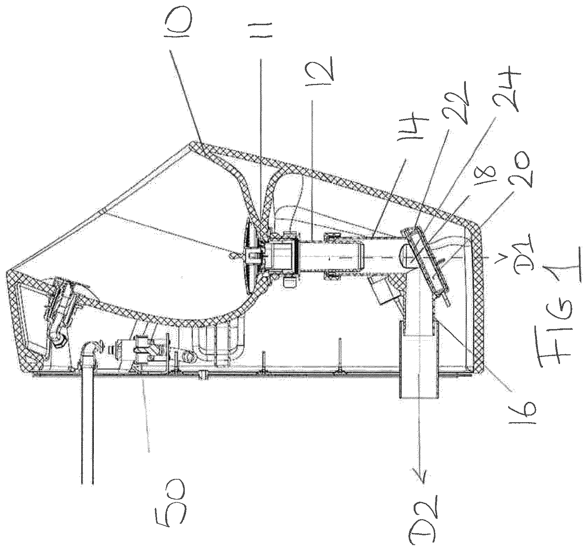

With reference to Figure 1, there is shown a system comprising a sanitary ware product in the form of a urinal 10. The urinal 10 defines an outlet 11 leading to a top end of an outlet pipe 12. The outlet pipe 12 is formed of three sections in the form of an upstream pipe section 14 extending from the outlet 12 in a first direction D1 and a downstream pipe section 16 extending in a second direction D2 different from the first direction D1, and an interface pipe section 18 located at the interface of the upstream and downstream pipe sections 14;16.

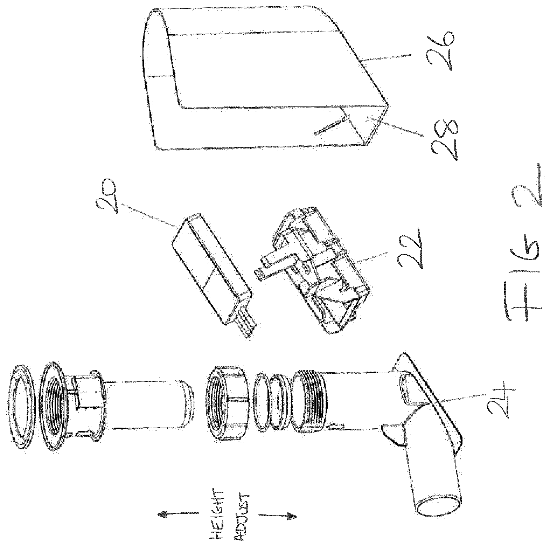

The system comprises a sensor 20 which is operable to detect the presence of fluid in the outlet pipe 12. The sensor is in contact with the interface pipe section 18, and is attached to the outlet pipe 12. The sensor 20 is best shown with reference to Figure 2 which shows a casing 22 which is attachable to the outlet pipe 12, wherein the sensor 20 is located inside the casing 22, along with a battery (not shown in the Figures) for powering the sensor. In one embodiment, the casing may be made of a plastic such as ABS. The sensor itself may take the form of a non-contact sensor, such as an inductive sensor, or a capacitive sensor, for detecting fluid through the outlet pipe.. In another embodiment, the sensor 20 may take the form of a pressure sensor which is operable to detect the presence of fluid in the outlet pipe based on fluid contacting a portion of the outlet pipe which the sensor is connected to. In the embodiment shown in Figure 1, that portion of the outlet pipe 12 is a flat surface 24 from the interface pipe section 18 which the sensor 20 is in contact with. The flat surface 24 is located underneath the outlet 11 of the urinal 10 such that fluid (such as urine) passing out from the outlet 11 into the outlet pipe 12 falls onto the flat surface 24, and then runs off the flat surface 24 down towards the downstream pipe section 16 and then on into a drain (not shown in the Figures). In some embodiments, the flat surface 24 may be orientated at between twenty degrees and thirty degrees with respect to the horizontal. In other embodiments, the lower bound of this angular range may be five, ten, or fifteen degrees, as opposed to twenty degrees. The upper bound of this angular range may also be forty, forty five, fifty, sixty, seventy or eighty degrees, as opposed to thirty degrees. In one particular embodiment, the flat surface 24 may be orientated at twenty five degrees with respect to the horizontal. At such orientations, the flat surface is better orientated to prevent fluid pooling at the vicinity of the sensor.

To help secure the casing 22 with respect to the outlet pipe 12, the system may further comprise a shroud 26 which is configured to extend around a portion of the outlet pipe 12, and preferably such that the casing 22 is further configured to rest on a surface 28 of the shroud 26 for supporting the casing 22 on the shroud 26. It will be appreciated that a clip or any other attachment means may be used to attach the shroud to either the casing or the outlet pipe. In one embodiment, the shroud may be made of a plastic such as polypropylene.

The operation of the sensor 20 will now be described with reference to the Figures. In the embodiment shown in Figure 2, the sensor is placed into contact with the flat surface 24 from the interface pipe section 18 using the casing 22. The sensor then operates to detect the presence of fluid in the outlet pipe by detecting fluid which impinges on the flat surface 24. Since the sensor 20 is in contact with the flat surface 24, the sensor 20 is configured to detect the presence of fluid in the outlet pipe 12 passing through the outlet pipe. As previously discussed, the sensor 20 used to detect the fluid may be a non-contact sensor, or some other sensor such as a pressure sensor. Based on the detection of such fluid, the sensor 20 generates a first signal containing first information relating to the presence of fluid in the outlet pipe. Depending on the configuration of the sensor 20, the sensor may generate the first signal in a number of different ways. In exemplary embodiments, the sensor 20 may be configured to generate the first signal upon each detection of fluid on the flat surface; or generate a plurality of first signals at regular time intervals (e.g. a first signal generated every few seconds).

The sensor 20 is configured to output the first signal to a processor. The location of the processor may be within the casing 22 or at a location remote from the sensor 20. As required, the sensor 20 may be connected to a transmitter, or a transceiver, which may be located in the casing 22, for transmitting the first signal to the processor.

The processor is configured to process the first information from the first signal (or plurality of first signals) to generate a second signal, and output this second signal to a device.

In one particular embodiment, the processor is configured to process the first information from the first signals to generate second information relating to the frequency in which the sanitary ware product is used in a period of time, wherein the second signal is generated based on the second information. The second information has several applications. For instance, in a broad sense, the second information is indicative of the general usage of the urinal 10. In a particular embodiment, the second information can be indicative of how often the urinal is being used, and can be used to then determine how often flushing/cleaning or the urinal 10 should be carried out.

In another embodiment, the processor may be configured to process the first information from the first signals to derive second information relating to a period of time in which fluid is present in the outlet pipe, wherein the second signal is generated based on the second information. In some embodiments, the processor may determine a blockage in the outlet pipe 12 in the event a period of time in which fluid is present in the outlet pipe 12 exceeds a threshold amount of time, or in the event a predetermined number of consecutive first signals contain first information indicating the presence of fluid in the outlet pipe 12. In other embodiments, the processor may determine a leakage from the urinal 10 or the outlet pipe 12 based on the first information from the sensor 20.

The processor is configured to output the second signal to a device. The device could be one of a plurality of different objects/locations depending on the configuration of the processor and the system.

In some embodiments, the device may be a flush valve 50 from a urinal, such as the flush valve shown Figure 1, and the second signal may comprise an instruction for controlling the operation of the flush valve 50. In some embodiments, the instruction for controlling the operation of the flush valve 50 may be an instruction to perform a flushing operation using the flush valve, or an instruction to control the frequency of the operation of the flush valve 50. In some cases the instruction for controlling the operation of the flush valve 50 may be an instruction to prohibit the operation of the flush valve (for instance, in the event the processor determines a blockage or leakage in the outlet pipe based on the first information from the sensor 20).

In another embodiment, the processor may be configured to prevent the operation of the flush valve 50 at a predetermined period of time, or may be configured to prohibit the operation of the flush valve when the first information indicates the presence of fluid in the outlet pipe. These embodiments would be particularly suited for urinals used in forums such as stadiums, theatres, and cinemas, where use of the urinal may peak at particular times (e.g. during an interval, or at the end, of a game/performance). During such peak times, the configuration of the processor may prevent the operation of the flush valve during such times, and allow operation of the flush valve after the peak time has passed.

In the above embodiments therefore, the processor processes the first information from the sensor 20 to control the operation of the flush valve 50 depending on the usage/condition of the urinal.

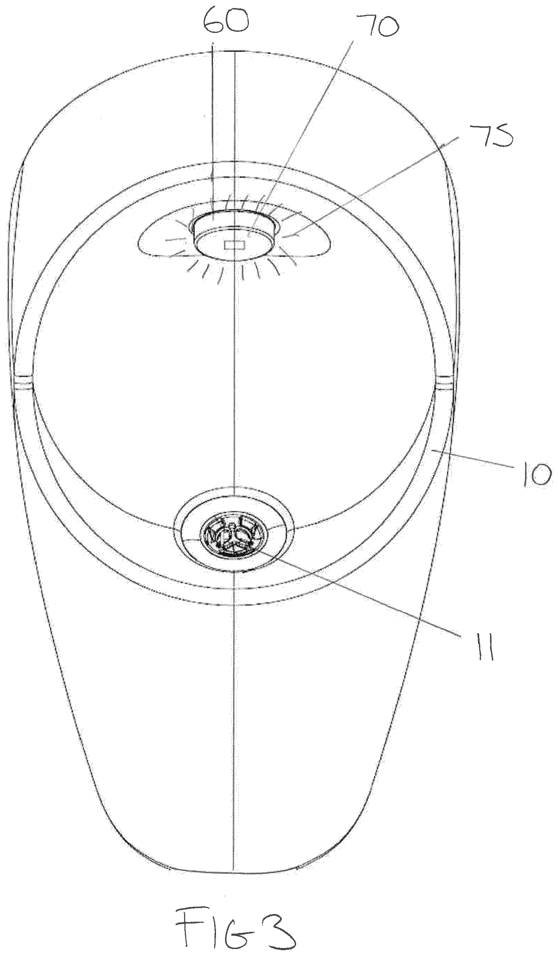

In further embodiments, the device may be a light source 60 from a urinal, such as the light source shown in the urinal of Figure 3, and the second signal may comprise an instruction for controlling the operation of the light source 60. It is envisaged in such embodiments that the light source 60 may be used to indicate a condition of the urinal and/or its outlet pipe. For instance, the light source may be configured to operate in a first condition during a normal, working, operation of the urinal 10. In some embodiments, the first condition may be the light source turned off, or emitting a first coloured light (e.g. green light). The light source may be configured to also operate in a second condition during a malfunction or a blockage, or a leakage, in the urinal 10. In some embodiments, the second condition may be the light source flashing, and/or emitting a second coloured light (e.g. red light) different from the first coloured light. In these embodiments, the processor processes the first information from the sensor to control the operation of the light source for indicating to a user the working state of the urinal. In the embodiments where the light source is configured to flash, the light source may comprise a first operation where the light source flashes with a first periodicity (e.g. one flash every second), and a second operation where the light source flashes with a second periodicity (e.g. one flash every five seconds) which is different from the first periodicity. The different set of flashes in the above embodiments, whilst at different periodicities, may be the same colour. In some embodiments, the different operations for the light source may be indicative of a different working state of the urinal.

In some embodiments, the instruction for controlling the operation of the flush valve 50 may be an instruction to perform a flushing operation using the flush valve, or an instruction to control the frequency of the operation of the flush valve 50.

With reference to the urinal shown in Figure 3, the urinal 10 also comprises a fluid outlet in the form of a spreader 70 from which cleaning fluid is emitted into the urinal 10. The spreader 70 is operably connected to the flush valve 50 for emitting cleaning fluid (such as water) into the urinal 10 during a flushing operation. In the particular embodiment of Figure 3, the spreader 70 comprises the light source 60, and light 75 from the light source 60 is configured to emanate from around the spreader 70 when the light source 60 is in use.

In terms of the second signal generated by the processor, in some embodiments this second signal may be output to a device which is remote from the sensor (a remote device) for sending and receiving information from the sensor, wherein the remote device is operable to send information to the sensor for controlling the operation of the sensor. Such a device may comprise at least one of a computer or a handheld device. In some embodiments, the device may be configured to send and receive information from a plurality of different sensors 20, each sensor attributed to a separate sanitary ware product.

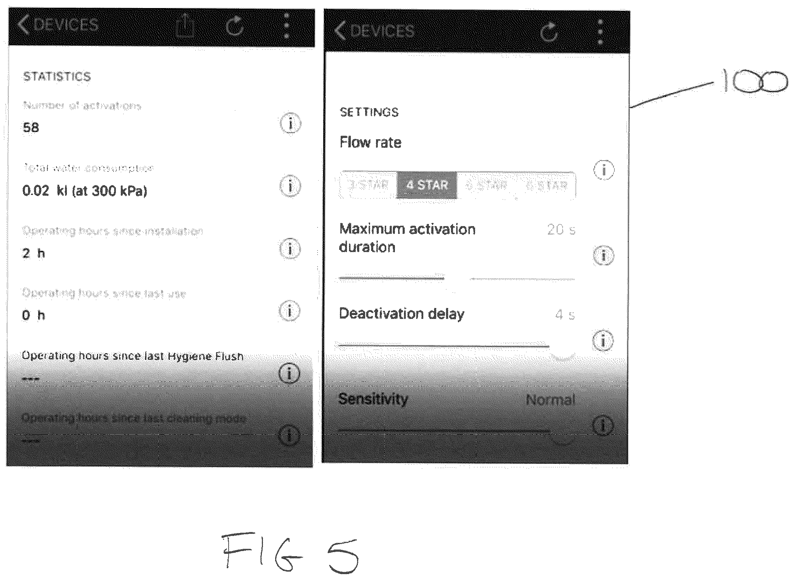

A user interface may be provided on the device for controlling the operation of the or each sensor 20 which the device is in communication with. An exemplary embodiment of such a user interface 100 is shown in Figure 5. The user interface may be configured to process the second information from each received second signal to output an operating condition or at least one usage statistic for the (each) urinal 10 to which the sensor 20 is attached. In some embodiments, the usage statistic may be the period of time since the sensor 20 was last activated, or the period of time since the sensor 20 was installed on the urinal 10.

The device is some embodiments may be configured to allow a user to control an operating parameter of the sensor 20 and/or an operating parameter of a flush valve 50 associated with the sensor 20. In some embodiments, the operating parameter may be the flow rate of cleaning fluid during a flushing operation, the duration of a flushing operation, a minimum period of time between flushing operations, and/or a sensitivity of the sensor 20.

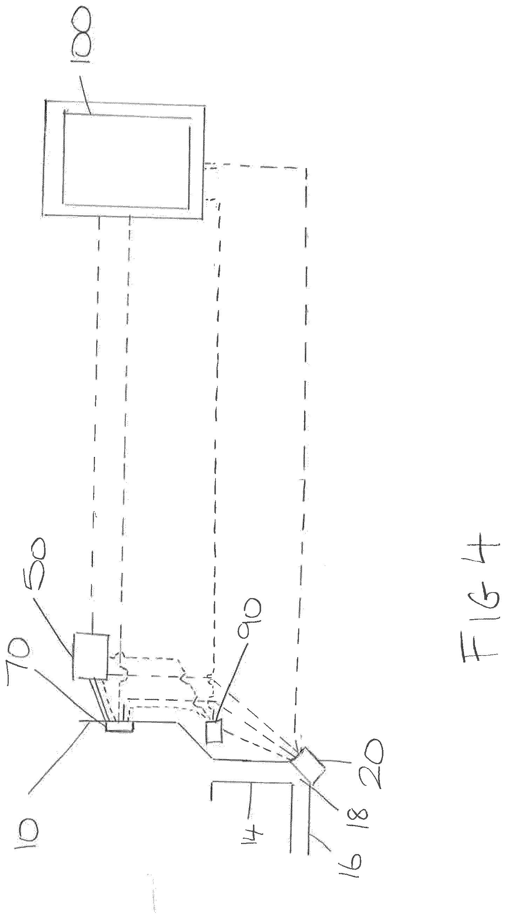

In some embodiments, depending on the type of sensor used, it is envisaged that the sensor 20 may be configured to detect a property of the fluid in the outlet pipe, for instance the salt levels in the fluid, or configured to detect a predetermined substance, or an amount thereof. This may be advantageous, particularly when the system comprises identification means 90 as shown in Figure 4 for identifying the user of the urinal (such as an RFID reader which is configured to read an RFID tag attached to the user of the urinal). In such arrangements, which are particularly suited to hospitals where patient monitoring is desirable, the processor may be configured to receive a third signal containing third information from the identification means 90, and process the third information along with the first information to generate the second signal. With this arrangement, the system effectively allows for the system to generate a second signal which can be used to indicate a status or condition of the user of the urinal based on the content of their urine (such as the concentration of the urine).

In terms of the location of the processor, with reference to Figure 4, it will be appreciated that it may be located anywhere in the system - for instance in the casing 22 next to the sensor 20, at the device, or some other remote location. As required, signals passed between any of the processor, the sensor 20, the battery, the flush valve 50, the light source 60, and the spreader 70, and the device may be transmitted using any appropriate communication means, including Bluetooth or other wireless communication means.

The above therefore describes various systems and methods which may be used to monitor blockages or leakages in the urinal and provide an indication of these malfunctions to a user or a party in charge of maintenance, and relates to method of using information taken from a sensor system associated with a urinal. In the event that a blockage is detected based on the information from the sensor system, the operation of a flush valve for the urinal may be prohibited to prevent flooding of the urinal.

It will be appreciated that various other modifications can be made to the systems and method described herein.

For instance, in one embodiment, to help assist the system being retrofitted to urinals of differing sizes having different length outlet pipes, the outlet pipe provided in the system may comprise a length of a portion of the outlet pipe which is adjustable. In the embodiment shown in Figure 2, the length of the upstream pipe section 14 is adjustable. For the sake of completeness, an intention of some embodiments of the system described herein is for the system to be retrofitting a sanitary ware product comprising an outlet pipe using the system.

In relation to the position of the sensor 20, in some embodiments the sensor may be positioned directly underneath the outlet 11 of the urinal 10, as shown in Figure 1, such to increase the effectiveness of the sensor at detecting small amounts of fluid passing out from the outlet 11 through the outlet pipe 12. To assist with the detection of fluid by the sensor 20, in some embodiments an end of the outlet pipe 12 proximal the outlet 11 may be provided with a flow restricting means, such as a reduction cone, for controlling the maximum flow rate of fluid passing through the outlet pipe 12 at any given time.

In some embodiments, the sensor may be powered by a mains electricity supply, with our without a corresponding battery.

Also in relation to the position of the sensor of the sensor 20, it will be appreciated that the sensor 20 need not expressly be located at the interface pipe section 18 of the outlet pipe 12, but may instead be positioned anywhere on or around the outlet pipe 12 such that the sensor 20 is operable to perform its function of detecting the presence of fluid in the outlet pipe 12. To that effect, in one embodiment, the sensor 20 may be in contact with an exterior surface of the outlet pipe 12.

It will also be appreciated the systems described herein, which have been described in some embodiments with reference to a urinal, may be used on any sanitary ware product comprising an outlet pipe, such as a bath, a shower, or a sink.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims, and that other embodiments may be utilised and modifications may be made without departing from the scope of the claimed invention as defined by the appended claims.

## Claims

1. A system comprising:
a sanitary ware product (10);
an outlet pipe (12) of the sanitary ware product (10);
a processor; and
a sensor (20) operable to detect the presence of fluid in the outlet pipe (12);
wherein the sensor (20) is configured to output to the processor at least one first signal containing first information relating to the presence of fluid in the outlet pipe (12); wherein the processor is configured to process the first information from the at least one first signal to generate a second signal, and output this second signal to a device;
wherein the sensor is attached to the outlet pipe; and
wherein the outlet pipe comprises an end located upstream of the sensor, wherein said end of the outlet pipe located upstream of the sensor (20) is connected to an outlet (11) of the sanitary ware product (10), and wherein the sensor (20) is positioned, in use of the system, directly underneath said end of the outlet pipe (12) located upstream of the sensor and directly underneath the outlet (11) of the sanitary ware product (10);
wherein the outlet pipe (12) comprises an upstream pipe section (14) extending in a first direction and a downstream pipe section (16) extending in a second direction different from the first direction, and an interface pipe section (18) located at the interface of the upstream and downstream pipe sections (14;16), wherein the sensor (20) is in contact with the interface pipe section (18);
**characterized in that** interface pipe section (18) comprises a flat surface (24), and wherein the sensor (20) is in contact with said flat surface (24), wherein said flat surface (24) is located underneath the outlet (11) of the sanitary ware product (10) such that fluid passing out from the outlet (11) of the sanitary ware product (10) into the outlet pipe (12) falls onto the flat surface (24), and then runs off the flat surface (24) down towards the downstream pipe section (16).

2. A system according to claim 1, wherein the sensor (20) is operable to detect the presence of fluid in the outlet pipe (12) based on fluid contacting a portion of the outlet pipe (12) which the sensor (20) is connected to.

3. A system according to claim 1 or 2, wherein the sensor (20) is a non-contact sensor.

4. A system according to any preceding claim, wherein the at least one first signal comprises a plurality of first signals, each first signal containing first information relating to the presence of fluid in the outlet pipe at a different point in time, wherein the processor is configured to process the first information from the plurality of first signals to generate the second signal.

5. A system according to claim 4, wherein the processor is configured to process the first information from the plurality of first signals to generate second information relating to the frequency in which the sanitary ware product is used in a period of time, wherein the second signal is generated based on the second information.

6. A system according to claim 4, wherein the processor is configured to process the first information from the plurality of first signals to derive second information relating to a period of time in which fluid is present in the outlet pipe, wherein the second signal is generated based on the second information, wherein the processor is configured to determine a blockage in the event the period of time exceeds a threshold amount of time.

7. A system according to any preceding claim, wherein the second signal comprises an instruction for controlling the operation of either a light source (60) or a flush valve (50) from the sanitary ware product (10).

8. A system according to any preceding claim, further comprising a casing (22) which is attachable to the outlet pipe (12) of the sanitary ware product (10), wherein the sensor (20) is located inside the casing (22).

9. A system according to claim 8, further comprising a shroud (26) which is configured to extend around a portion of the outlet pipe (12) of a sanitary ware product (10), wherein the casing (22) is configured to rest on a surface of the shroud (26) for supporting the casing (22) on the shroud (26).

10. A system according to any preceding claim, further comprising the device, wherein the device is located at a remote location from the sensor (20) for sending and receiving information from the sensor (20), wherein the remote location is further operable to send information to the sensor (20) for controlling the operation of the sensor (20).

11. A system according to any preceding claim, further comprising the device, wherein the device is at least one of:
i) a computer or a handheld device;
ii) a flush valve (50); or
iii) a light source (60) from the sanitary ware product.

12. A system according to any preceding claim, wherein the sensor (20) is in contact with an exterior surface of the outlet pipe (12).

13. A system according to any preceding claim, wherein the flat surface, in use of the system, is orientated at between twenty degrees and thirty degrees with respect to the horizontal.

14. A system according to any preceding claim, wherein a length of a portion of the outlet pipe (12) is adjustable.

15. A system according to any preceding claim wherein the sanitary ware product (10) is a urinal.

16. A method for monitoring the use of a sanitary ware product (10) comprising an outlet pipe (12), using a system comprising a processor and a sensor (20) operable to detect the presence of fluid in the outlet pipe (12); wherein the method comprises the steps of:
using the sensor (20) to output to the processor at least one first signal containing first information relating to the presence of fluid in the outlet pipe (12); and
using the processor to process the first information from the at least one first signal to generate a second signal;
wherein the sensor (20) is attached to the outlet pipe (12); and
wherein the outlet pipe (12) comprises an end located upstream of the sensor (20), wherein said end of the outlet pipe (12) located upstream of the sensor (20) is connectable to an outlet (11) of the sanitary ware product, and wherein the sensor (20) is positioned, in use, directly underneath said end of the outlet pipe (12) located upstream of the sensor (20) and directly underneath the outlet (11) of the sanitary ware product (10);
wherein the outlet pipe (12) comprises an upstream pipe section (14) extending in a first direction and a downstream pipe section (16) extending in a second direction different from the first direction, and an interface pipe section (18) located at the interface of the upstream and downstream pipe sections (14;16), wherein the sensor (20) is in contact with the interface pipe section (18);
**characterized in that** interface pipe section (18) comprises a flat surface (24), and wherein the sensor is in contact with said flat surface (24), wherein said flat surface (24) is located underneath the outlet (11) of the sanitary ware product (10) such that fluid passing out from the outlet (11) of the sanitary ware product (10) into the outlet pipe (12) falls onto the flat surface (24), and then runs off the flat surface (24) down towards the downstream pipe section (16).

## Patentansprüche

1. System, umfassend:
ein Sanitärwarenprodukt (10);
ein Auslassrohr (12) des Sanitärwarenprodukts (10);
einen Prozessor; und
einen Sensor (20), der dazu betreibbar ist, das Vorhandensein von Flüssigkeit in dem Auslassrohr (12) zu detektieren;
wobei der Sensor (20) dafür ausgelegt ist, an den Prozessor wenigstens ein erstes Signal auszugeben, das erste Informationen zu dem Vorhandensein von Flüssigkeit in dem Auslassrohr (12) enthält;
wobei der Prozessor dafür ausgelegt ist, die ersten Informationen aus dem wenigstens einen ersten Signal zu verarbeiten, um ein zweites Signal zu generieren, und dieses zweite Signal an eine Vorrichtung auszugeben;
wobei der Sensor an dem Auslassrohr angebracht ist; und
wobei das Auslassrohr ein Ende umfasst, das sich stromaufwärts des Sensors befindet, wobei das Ende des Auslassrohrs, welches sich stromaufwärts des Sensors (20) befindet, mit einem Auslass (11) des Sanitärwarenprodukts (10) verbunden ist, und wobei der Sensor (20), bei Verwendung des Systems, direkt unter dem Ende des Auslassrohrs (12) angeordnet ist, welches sich stromaufwärts des Sensors befindet, und direkt unter dem Auslass (11) des Sanitärwarenprodukts (10);
wobei das Auslassrohr (12) umfasst: einen stromaufwärts angeordneten Rohrabschnitt (14), der sich in einer ersten Richtung erstreckt, und einen stromabwärts angeordneten Rohrabschnitt (16), der sich in einer zweiten Richtung erstreckt, die von der ersten Richtung verschieden ist,
und einen zwischengeschalteten Rohrabschnitt (18), der sich an der Schnittstelle des stromaufwärts angeordneten und des stromabwärts angeordneten Rohrabschnitts (14; 16) befindet, wobei der Sensor (20) in Kontakt mit dem zwischengeschalteten Rohrabschnitt (18) steht;
**dadurch gekennzeichnet, dass** der zwischengeschaltete Rohrabschnitt (18) eine flache Fläche umfasst (24), und wobei der Sensor (20) in Kontakt mit der flachen Fläche (24) steht, wobei sich die flache Fläche (24) unter dem Auslass (11) des Sanitärwarenprodukts (10) befindet, sodass Flüssigkeit, die von dem Auslass (11) des Sanitärwarenprodukts (10) aus- und in das Auslassrohr (12) eintritt, auf die flache Fläche (24) fällt und dann an der flachen Fläche (24) in Richtung des stromabwärts angeordneten Rohrabschnitts (16) herunterläuft.

2. System gemäß Anspruch 1, wobei der Sensor (20) dazu betreibbar ist, das Vorhandensein von Flüssigkeit in dem Auslassrohr (12) basierend darauf zu detektieren, dass Flüssigkeit einen Abschnitt des Auslassrohrs (12) berührt, mit dem der Sensor (20) verbunden ist.

3. System gemäß Anspruch 1 oder 2, wobei der Sensor (20) ein kontaktloser Sensor ist.

4. System gemäß einem der vorstehenden Ansprüche, wobei das wenigstens eine erste Signal mehrere erste Signale umfasst, wobei jedes erste Signal erste Informationen zu dem Vorhandensein von Flüssigkeit in dem Auslassrohr zu einem anderen Zeitpunkt enthält, wobei der Prozessor dafür ausgelegt ist, die ersten Informationen aus den mehreren ersten Signalen zu verarbeiten, um das zweite Signal zu generieren.

5. System gemäß Anspruch 4, wobei der Prozessor dafür ausgelegt ist, die ersten Informationen aus den mehreren ersten Signalen zu verarbeiten, um zweite Informationen zu der Häufigkeit zu generieren, mit der das Sanitärwarenprodukt in einem Zeitraum verwendet wird, wobei das zweite Signal basierend auf den zweiten Informationen generiert wird.

6. System gemäß Anspruch 4, wobei der Prozessor dafür ausgelegt ist, die ersten Informationen aus den mehreren ersten Signalen zu verarbeiten, um zweite Informationen zu einem Zeitraum abzuleiten, in dem Flüssigkeit in dem Auslassrohr vorhanden ist, wobei das zweite Signal basierend auf den zweiten Informationen generiert wird, wobei der Prozessor dafür ausgelegt ist, eine Blockierung zu bestimmen, für den Fall, dass der Zeitraum eine Schwellwert-Zeitdauer überschreitet.

7. System gemäß einem der vorstehenden Ansprüche, wobei das zweite Signal eine Anweisung zum Steuern des Betriebs einer Lichtquelle (60) oder eines Spülventils (50) von dem Sanitärwarenprodukt (10) umfasst.

8. System gemäß einem der vorstehenden Ansprüche, ferner umfassend ein Gehäuse (22), das an dem Auslassrohr (12) des Sanitärwarenprodukts (10) angebracht werden kann, wobei sich der Sensor (20) im Inneren des Gehäuses (22) befindet.

9. System gemäß Anspruch 8, ferner umfassend eine Ummantelung (26), die dafür ausgelegt ist, sich um einen Abschnitt des Auslassrohrs (12) eines Sanitärwarenprodukts (10) zu erstrecken, wobei das Gehäuse (22) dafür ausgelegt ist, auf einer Fläche der Ummantelung (26) zu ruhen, um das Gehäuse (22) auf der Ummantelung (26) zu stützen.

10. System gemäß einem der vorstehenden Ansprüche, ferner umfassend die Vorrichtung, wobei sich die Vorrichtung an einem vom Sensor (20) entfernten Ort zum Senden und Empfangen von Informationen an den/von dem Sensor (20) befindet, wobei der entfernte Ort ferner dazu betreibbar ist, Informationen an den Sensor (20) zu senden, um den Betrieb des Sensors (20) zu steuern.

11. System gemäß einem der vorstehenden Ansprüche, ferner umfassend die Vorrichtung, wobei die Vorrichtung wenigstens eines hiervon ist:
i) ein Computer oder eine handgehaltene Vorrichtung;
ii) ein Spülventil (50); oder
iii) eine Lichtquelle (60) von dem Sanitärwarenprodukt.

12. System gemäß einem der vorstehenden Ansprüche, wobei der Sensor (20) in Kontakt mit einer Außenfläche des Auslassrohrs (12) steht.

13. System gemäß einem der vorstehenden Ansprüche, wobei die flache Fläche bei Verwendung des Systems zwischen zwanzig Grad und dreißig Grad in Bezug auf die Horizontale ausgerichtet ist.

14. System gemäß einem der vorstehenden Ansprüche, wobei eine Länge eines Abschnitts des Auslassrohrs (12) anpassbar ist.

15. System gemäß einem der vorstehenden Ansprüche, wobei das Sanitärwarenprodukt (10) ein Urinal ist.

16. Verfahren zum Überwachen der Verwendung eines Sanitärwarenprodukts (10), das ein Auslassrohr (12) umfasst, wobei ein System verwendet wird, das einen Prozessor und einen Sensor (20) umfasst, der dazu betreibbar ist, das Vorhandensein von Flüssigkeit in dem Auslassrohr (12) zu detektieren, wobei das Verfahren diese Schritte umfasst:
Verwenden des Sensors (20), um an den Prozessor wenigstens ein erstes Signal auszugeben, das erste Informationen zu dem Vorhandensein von Flüssigkeit in dem Auslassrohr (12) enthält; und
Verwenden des Prozessors, um die ersten Informationen aus dem wenigstens einen ersten Signal zu verarbeiten, um ein zweites Signal zu generieren;
wobei der Sensor (20) an dem Auslassrohr (12) angebracht ist; und
wobei das Auslassrohr (12) ein Ende umfasst, das sich stromaufwärts des Sensors (20) befindet, wobei das Ende des Auslassrohrs (12), welches sich stromaufwärts des Sensors (20) befindet, mit einem Auslass (11) des Sanitärwarenprodukts verbunden werden kann, und wobei der Sensor (20), bei Verwendung, direkt unter dem Ende des Auslassrohrs (12) angeordnet ist, welches sich stromaufwärts des Sensors (20) befindet, und direkt unter dem Auslass (11) des Sanitärwarenprodukts (10);
wobei das Auslassrohr (12) umfasst: einen stromaufwärts angeordneten Rohrabschnitt (14), der sich in einer ersten Richtung erstreckt, und einen stromabwärts angeordneten Rohrabschnitt (16), der sich in einer zweiten Richtung erstreckt, die von der ersten Richtung verschieden ist, und einen zwischengeschalteten Rohrabschnitt (18), der sich an der Schnittstelle des stromaufwärts angeordneten und des stromabwärts angeordneten Rohrabschnitts (14; 16) befindet, wobei der Sensor (20) in Kontakt mit dem zwischengeschalteten Rohrabschnitt (18) steht;
**dadurch gekennzeichnet, dass** der zwischengeschaltete Rohrabschnitt (18) eine flache Fläche umfasst (24), und wobei der Sensor in Kontakt mit der flachen Fläche (24) steht, wobei sich die flache Fläche (24) unter dem Auslass (11) des Sanitärwarenprodukts (10) befindet, sodass Flüssigkeit, die von dem Auslass (11) des Sanitärwarenprodukts (10) aus- und in das Auslassrohr (12) eintritt, auf die flache Fläche (24) fällt und dann an der flachen Fläche (24) in Richtung des stromabwärts angeordneten Rohrabschnitts (16) herunterläuft.

## Revendications

1. Système comprenant :
un produit d'équipement sanitaire (10) ;
un tuyau de sortie (12) du produit d'équipement sanitaire (10) ;
un processeur ; et
un capteur (20) pouvant fonctionner pour détecter la présence de fluide dans le tuyau de sortie (12) ;
dans lequel le capteur (20) est configuré pour sortir vers le processeur au moins un premier signal contenant des premières informations relatives à la présence de fluide dans le tuyau de sortie (12) ; dans lequel le processeur est configuré pour traiter les premières informations provenant de l'au moins un premier signal pour générer un second signal, et sortir ce second signal vers un dispositif ;
dans lequel le capteur est fixé au tuyau de sortie ; et
dans lequel le tuyau de sortie comprend une extrémité située en amont du capteur, dans lequel ladite extrémité du tuyau de sortie située en amont du capteur (20) est connectée à une sortie (11) du produit d'équipement sanitaire (10), et dans lequel le capteur (20) est positionné, lors de l'utilisation du système, directement sous ladite extrémité du tuyau de sortie (12) située en amont du capteur et directement sous la sortie (11) du produit d'équipement sanitaire (10) ;
dans lequel le tuyau de sortie (12) comprend une section de tuyau amont (14) s'étendant dans une première direction et une section de tuyau aval (16) s'étendant dans une seconde direction différente de la première direction, et une section de tuyau d'interface (18) située au niveau de l'interface des sections de tuyau amont et aval (14 ; 16), dans lequel le capteur (20) est en contact avec la section de tuyau d'interface (18) ;
**caractérisé en ce que** la section de tuyau d'interface (18) comprend une surface plate (24), et dans lequel le capteur (20) est en contact avec ladite surface plate (24), dans lequel ladite surface plate (24) est située sous la sortie (11) du produit d'équipement sanitaire (10) de telle sorte que le fluide sortant de la sortie (11) du produit d'équipement sanitaire (10) dans le tuyau de sortie (12) tombe sur la surface plate (24), et s'écoule ensuite de la surface plate (24) vers le bas vers la section de tuyau en aval (16).

2. Système selon la revendication 1, dans lequel le capteur (20) est utilisable pour détecter la présence de fluide dans le tuyau de sortie (12) sur la base du contact du fluide avec une partie du tuyau de sortie (12) à laquelle est connecté le capteur (20).

3. Système selon la revendication 1 ou 2, dans lequel le capteur (20) est un capteur sans contact.

4. Système selon l'une quelconque revendication précédente, dans lequel l'au moins un premier signal comprend une pluralité de premiers signaux, chaque premier signal contenant des premières informations relatives à la présence de fluide dans le tuyau de sortie à un instant différent, dans lequel le processeur est configuré pour traiter les premières informations provenant de la pluralité de premiers signaux pour générer le second signal.

5. Système selon la revendication 4, dans lequel le processeur est configuré pour traiter les premières informations provenant de la pluralité de premiers signaux pour générer des secondes informations relatives à la fréquence à laquelle le produit d'équipement sanitaire est utilisé dans une période de temps, dans lequel le second signal est généré sur la base des secondes informations.

6. Système selon la revendication 4, dans lequel le processeur est configuré pour traiter les premières informations provenant de la pluralité de premiers signaux pour dériver des secondes informations relatives à une période de temps pendant laquelle le fluide est présent dans le tuyau de sortie, dans lequel le second signal est généré sur la base des secondes informations, dans lequel le processeur est configuré pour déterminer un blocage dans le cas où la période de temps dépasse une quantité de temps seuil.

7. Système selon l'une quelconque revendication précédente, dans lequel le second signal comprend une instruction pour commander le fonctionnement soit d'une source de lumière (60), soit d'une vanne de chasse d'eau (50) du produit d'équipement sanitaire (10).

8. Système selon l'une quelconque revendication précédente, comprenant en outre un boîtier (22) qui peut être fixé au tuyau de sortie (12) du produit d'équipement sanitaire (10), dans lequel le capteur (20) est situé à l'intérieur du boîtier (22).

9. Système selon la revendication 8, comprenant en outre une enveloppe (26) qui est configurée pour s'étendre autour d'une partie du tuyau de sortie (12) d'un produit d'équipement sanitaire (10), dans lequel le boîtier (22) est configuré pour reposer sur une surface de l'enveloppe (26) pour supporter le boîtier (22) sur l'enveloppe (26).

10. Système selon l'une quelconque revendication précédente, comprenant en outre le dispositif, dans lequel le dispositif est situé au niveau d'un emplacement distant du capteur (20) pour envoyer et recevoir des informations du capteur (20), dans lequel l'emplacement distant peut en outre fonctionner pour envoyer des informations au capteur (20) pour commander le fonctionnement du capteur (20).

11. Système selon l'une quelconque revendication précédente, comprenant en outre le dispositif, dans lequel le dispositif est au moins un parmi :
i) un ordinateur ou un dispositif portatif ;
ii) une vanne de chasse d'eau (50) ; ou
iii) une source de lumière (60) provenant du produit d'équipement sanitaire.

12. Système selon l'une quelconque revendication précédente, dans lequel le capteur (20) est en contact avec une surface extérieure du tuyau de sortie (12).

13. Système selon l'une quelconque revendication précédente, dans lequel la surface plate, lors de l'utilisation du système, est orientée entre vingt degrés et trente degrés par rapport à l'horizontale.

14. Système selon l'une quelconque revendication précédente, dans lequel une longueur d'une partie du tuyau de sortie (12) est réglable.

15. Système selon l'une quelconque revendication précédente, dans lequel le produit d'équipement sanitaire (10) est un urinoir.

16. Procédé pour surveiller l'utilisation d'un produit d'équipement sanitaire (10) comprenant un tuyau de sortie (12), utilisant un système comprenant un processeur et un capteur (20) pouvant fonctionner pour détecter la présence de fluide dans le tuyau de sortie (12) ; dans lequel le procédé comprend les étapes suivantes :
l'utilisation du capteur (20) pour sortir vers le processeur au moins un premier signal contenant des premières informations relatives à la présence de fluide dans le tuyau de sortie (12) ; et
l'utilisation du processeur pour traiter les premières informations provenant de l'au moins un premier signal pour générer un second signal ;
dans lequel le capteur (20) est fixé au tuyau de sortie (12) ; et
dans lequel le tuyau de sortie (12) comprend une extrémité située en amont du capteur (20), dans lequel ladite extrémité du tuyau de sortie (12) située en amont du capteur (20) peut être raccordée à une sortie (11) du produit d'équipement sanitaire, et dans lequel le capteur (20) est positionné, lors de l'utilisation, directement sous ladite extrémité du tuyau de sortie (12) située en amont du capteur (20) et directement sous la sortie (11) du produit d'équipement sanitaire (10) ;
dans lequel le tuyau de sortie (12) comprend une section de tuyau amont (14) s'étendant dans une première direction et une section de tuyau aval (16) s'étendant dans une seconde direction différente de la première direction, et une section de tuyau d'interface (18) située au niveau de l'interface des sections de tuyau amont et aval (14 ; 16), dans lequel le capteur (20) est en contact avec la section de tuyau d'interface (18) ;
**caractérisé en ce que** la section de tuyau d'interface (18) comprend une surface plate (24), et dans lequel le capteur est en contact avec ladite surface plate (24), dans lequel ladite surface plate (24) est située sous la sortie (11) du produit d'équipement sanitaire (10) de telle sorte que le fluide sortant par la sortie (11) du produit d'équipement sanitaire (10) dans le tuyau de sortie (12) tombe sur la surface plate (24), et s'écoule ensuite de la surface plate (24) vers le bas vers la section de tuyau en aval (16).
